# EUROPEAN PATENT APPLICATION

(11) **EP 2 243 417 A1**
(43) Date of publication of application: **27.10.2010**
(21) Application number: 09707188.0
(22) Date of filing: 04.02.2009
(51) Int. Cl.: A61B 1/00

(54) **METHOD AND DEVICE FOR AUTOMATED TRANSLATIONAL MOVEMENT OF AN ENDOSCOPE THROUGH THE DIGESTIVE TRACT**

(30) Priority: 04.02.2008 ES 200800391
(71) Applicant: Universidad Politecnica De Valencia, 46022 Valencia (ES); Fundación Para La Investigación Hospital La Fé, 46009 Valencia (ES)
(72) Inventor: JORDÁ ALBIÑANA, María Begoña, E-46022 Valencia (ES); PONS BELTRÁN, Vicente, E-46009 Valencia (ES); SALAS FELIS, Teresa, E-46009 Valencia (ES); SÁNCHEZ DÍAZ, Carlos, E-46022 Valencia (ES); SANTONJA GIMENO, Alberto Vicente, E-46022 Valencia (ES); SONGEL GONZÁLEZ, Gabriel, E-46022 Valencia (ES)
(74) Representative: Pons Ariño, Angel
(86) International application number: PCT/ES2009/000062
(87) International publication number: WO 2009/098337

(57) **Abstract**

Device for automated translational movement of an endoscope through the digestive tract, comprising, at least: (i) pneumatic translational movement means (1); and (ii) electronic control means (2), that enable the automated and coordinated method of the translational movement means (1). The translational movement means (1) include: at least one radial chamber immobilized on the endoscope; at least one bellows-shaped axial chamber connected via one end to the body of the endoscope; at least one radial chamber floating on the endoscope and secured to the second end of the bellows-shaped axial chamber.

## Description

### OBJECT OF THE INVENTION

The object of the present invention is formed by a method and a device for automated translational movement of an endoscope inside the digestive tract, said method and device based on the use of inflatable cavities which can be adapted to the endoscope and to the digestive tract wherethrough it has to pass. The device comprises a plurality of inflatable cavities which, with a coordinated movement, manages to retract the small intestine or digestive tract on the endoscope, by a movement control, based on the use of a pneumatic system controlled by an electronic system directed by microcontroller.

The present invention relates to a medical instrument suitable to be inserted inside the human body.

### ANTECEDENTES OF THE INVENTION

In the field of clinical gastroenterology, digestive tract pathologies occupy the most important volume. From the oesophagus to the rectum there are over 10 metres of digestive tract where a multitude of pathologies can occur. Access to said digestive tract in a minimally invasive manner, making use of the natural orifices, mouth and anus, has been vital for the diagnosis and handling of this large group of diseases.

The development of digestive endoscopy has facilitated said access and enabled the development of therapeutic actions otherwise unimaginable. There have been many advances in the last 30 years in the field of digestive endoscopy which has made it possible to effectively act in the most proximal and distal portions of the digestive tract.

Upper endoscopy or gastroscopy makes it possible to reach without any difficulties and with good acceptance by the patient the proximal structures of the digestive tract: oesophagus, stomach and first and second duodenal portion. This has led to the development of real minimally invasive endoscopic surgery.

Something similar occurs with the colon, the sigmoid colon and the rectum which are the most distal portions of the digestive tract using colonoscopy. Even with this same technique it is possible to skilfully reach the most distal portion of the small intestine, the terminal ileum.

There has always been a area of the digestive tract which, on the other hand is the longest (around 7 metres) that is difficult to access both anterograde (mouth) and retrograde (anal). Since a few years back the access to this portion has been facilitated with the appearance of "double balloon enteroscopy". It is a device similar to the gastroscope, of greater length and equipped with two balloons and an overtube which, by drive and retraction movements enable the advance of the endoscope through the small intestine. Although this technique has meant an important advance in the diagnosis and treatment of small intestine diseases, it continues to be an endoscopic method that requires significant skill, with considerable exploration time, collaboration with an anaesthetist and considerable discomfort for the patient. In recent months other models have come onto the market but strictly based on this initial model of the double balloon and with the same difficulties and drawbacks as it.

Application PCT WO01/08548 discloses a self-propelled device for its insertion in a passage or tube which has retractile walls, where said self-propelled devices comprises a body and at least retractile means to stimulate the walls and be able to move forwards, these means being electrically stimulated.

European patent application EP-1726250 relates to a double-balloon endoscope system, formed by two concentric tubes, at the end whereof there are different inflatable balloons. By inflating one or another of the balloons, the intestinal tract is widened, the time used to make the tube, via the end with the balloon not inflated at that time, move forward. With this movement between both concentric tubes, rhythmic with the inflation or deflation of the balloons, the endoscope is made to advance through the intestinal tract. This simple movement *a priori* involves certain risks due to uncontrolled advance within the intestinal tract and the lack of knowledge of how the intestine walls are constituted, for which reason said system and method involve numerous difficulties and drawbacks which make its execution problematic.

### DESCRIPTION OF THE INVENTION

To mitigate the aforementioned problems, the system is presented for automated translational movement based on inflatable cavities which can be adapted to the endoscope, object of the present patent application.

The device of the invention comprises, at least:
(i) translational movement means, preferably pneumatic, comprising in turn a plurality of latex cavities which, on expanding and contracting in coordinated manner, achieve different movement effects, whose dimensions are proportional to the calibre of the endoscope, whereon they will be placed, as well as the calibre and texture of the intestine, which they should retract; and
(ii) electronic control means, that enable the automated and coordinated method of the translational movement means, also comprising:
   a. logical and programmable control means;
   b. means of actuation on the translational movement means, which enable the activation and deactivation of the translational movement means;
   c. pressure collection means;
   d. control means of the translational movement means; and
   e. man-machine interface means.

By means of this system thus described, a solution is achieved to the need for a system which allows easier and more effective access to the small intestine. Therefore, a system is designed which is capable, by adapting to a conventional endoscope, of retracting the intestine on the endoscope, indirectly facilitating its advance.

The translational movement means is formed, as previously stated by a plurality of independent inflatable latex cavities, which contract and expand on introducing a fluid, which can be air, under pressure therein, distinguishing between two types of chambers, some of radial expansion with toroidal shape which adjust to the walls of the intestine or digestive tract in general and a second type of bellows-shaped axial expansion chamber, whose movement displaces one of the floating toroidal chambers incorporated in the endoscope and with it manages to make said endoscope advance through the small intestine or digestive tract in general.

The endoscope and, to achieve its advance through the inside of the intestine, has:
- at least one radial expansion chamber immobilized on the endoscope, although a preferred embodiment will have an additional toroidal chamber secured to the end of the endoscope.
- At least one bellows-shaped axial expansion chamber connected via one end to the body of the endoscope
- At least one toroidal radial expansion chamber floating on the endoscope and secured to the second end of the bellows-shaped axial chamber.

When the chambers described in the invention are indicated as radial or axial expansion chambers it means that said chambers, by the inclusion of any fluid, although preferably it can be pneumatic actuation through air or gas or by hydraulic action via any liquid fluid, achieving its enlargement or expansion in radial or axial direction with respect to the endoscope body, so when it states axial expansion it will be in the direction of the axis of the endoscope whilst radial direction will be in the direction of radius of said endoscope.

When throughout the specification it specifies in general the use through the digestive tract of said endoscope, this refers to the oesophagus, stomach and the whole small intestine and the rectum and large intestine until covering all the small intestine. This device will be very advisable, for example, in the advance of the endoscope through the small intestine due to the fact that said tract is of greater length and more tortuous path, which may further hinder the passage of the endoscope therethrough. For this reason, throughout the specification we speak of insertion of the endoscope through the small intestine but this does not limit its use to any part of the digestive tract.

The harmonized and controlled movement of these chambers in their inflation and deflation by the incorporation of electronic means manages to retract the intestine and indirectly make the endoscope advance inside it. The method for automated translational movement of the endoscope of the invention with the following stages:
A - inserting the endoscope in the digestive tract.
B - Inflation of the radial expansion chamber floating on the endoscope to adjust to the digestive tract.
C - Inflation of the axial expansion chamber and corresponding displacement of the floating radial expansion chamber together with the digestive tract whereto it was fixed
D - Inflation of the radial expansion chamber fixed to the endoscope for its fixing to the digestive tract
E - Deflation of the floating radial expansion chamber with the subsequent retraction of the axial expansion chamber and advance of the floating radial expansion chamber.
F - Inflation of the radial expansion chamber floating on the endoscope to adjust to the digestive tract.
G - Deflation of the radial expansion chamber fixed to the endoscope for its fixing to the digestive tract.

Back to stage C and successive to retract the intestine on the endoscope and indirectly make it advance through the inside thereof.

An improvement to this method will be found in the incorporation of an additional radial expansion chamber at the end of the endoscope that fixes the head thereof to the digestive tract and avoid that the retracted intestine returns to its natural position and loses the advance achieved.

It should be mentioned that the movement of the chambers that facilitates the retraction of the intestine, could even be programmed to carry out an inverse movement for expanding in the event of the need of the intestine or blocking thereof.

### BRIEF DESCRIPTION OF THE FIGURES

Below, a series of drawings is very briefly described that help to better understand the invention and are expressly related to an embodiment of said invention presented as a non-limitative example thereof.
Figure 1 represents the translational movement means that form part of the system object of the present invention, including a plan view (figure 1 a), a plan and sectional view (figure 1 b) and perspective view (figure 1 c)
Figure 2 represents a diagram of the electronic control means that form part of the system object of the present invention.
Figure 3 consists of a schematized representation of the movements of the translational movement means that form part of the system object of the present invention.

### DESCRIPTION OF A PREFERRED EMBODIMENT OF THE INVENTION

The system for automated translational movement based on inflatable cavities which can be adapted to an endoscope, object of the present invention patent. Said system comprising, at least:
(i) pneumatic translational movement means (1); and
(ii) electronic control means (2), which enable the automated and coordinated method of the translational movement means (1).

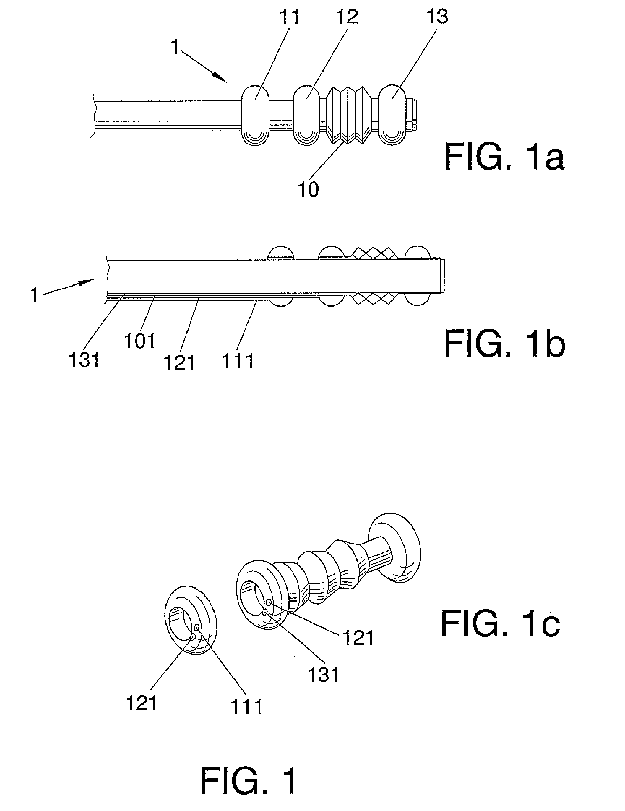

As can be observed in figure 1, the translational movement means (1) comprise at least, an axial expansion chamber (10) connected to a first tube (101), a radial expansion chamber (11) connected to a second tube (111), a floating radial expansion chamber (12) connected to a third tube (121) and an additional radial expansion chamber (13) connected to a fourth tube (131); all of this so that they can independently inflate said chambers via their respective tubes.

Both the radial expansion chambers (11), (12) and (13), and the axial expansion chamber (10) have a toroidal shape which surrounds the endoscope (3). The chambers (12) and (13) together with the axial expansion chamber (10) are solidly joined, whilst they are separately inflated. The first radial expansion chamber (11) is independent from the rest of the assembly maintaining a distance between 0 and 10 mm, depending on the state of the axial expansion chamber (10), in other words, it if is inflated or deflated. This movement, together with the inflation of the chambers to secure the intestine (4) is what makes retraction of the intestine possible and with this the advance of the endoscope (3) for exploration therein.

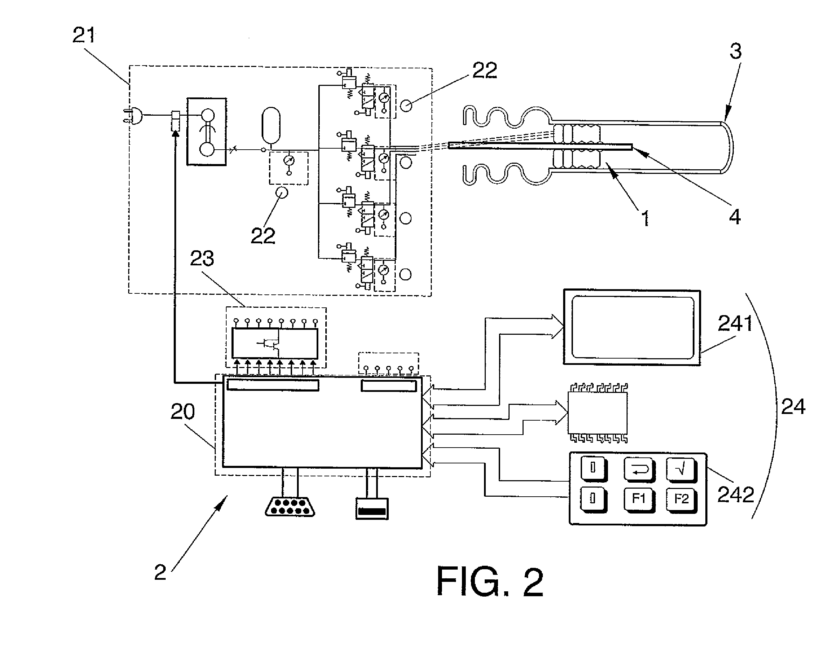

Said translational movement means (1) are controlled by the electronic control means (2), as observed in figure 2. Said electronic control means (2) comprise, at least:
- programmable control means (20), in this practical embodiment a microcontroller;
- means of actuation (21) on the translational movement means (1), which enable the activation and deactivation of said translational movement means (1), comprising a plurality of electrovalves;
- pressure collecting means (22);
- control means (23) of the translational movement means (1), so that the outputs of the programmable control means (20) adapt, activating and deactivating the different electrovalves that form the means of actuation (21); and
- man-machine interface means (24) comprising a data display screen (241) and a keyboard (242).

Thanks to the activation and deactivation of the electrovalves of the control means (23) a movement sequence is established.

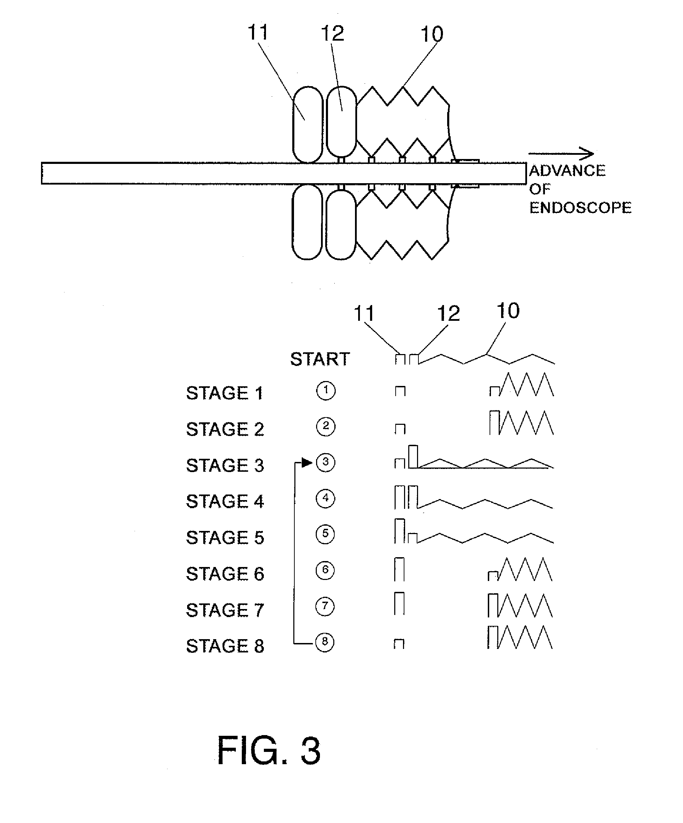

Figure 3 shows a schematized representation of the movements of the translational movement means that form part of the system object of the present invention. To explain the advance movement of the endoscope, reference to the additional radial expansion chamber (13) included in figure 1 has been removed.

The method for automated translational movement of the endoscope of the invention includes the following stages:
- Initial stage with the insertion of the endoscope in the digestive tract with both radial expansion chambers without pressure and with the axial expansion chamber distended to the maximum.
- Stage 1 retraction of the axial expansion chamber and, therefore, of the floating radial expansion chamber
- Stage 2 Inflation of the radial expansion chamber floating on the endoscope to adjust to the digestive tract.
- Stage 3 Inflation of the axial expansion chamber and corresponding displacement of the floating radial expansion chamber together with the digestive tract whereto it was fixed
- Stage 4 Inflation of the radial expansion chamber fixed to the endoscope for its fixing to the digestive tract
- Stage 5 Deflation of the floating radial expansion chamber and retraction of the axial expansion chamber with the consequent advance of the floating radial expansion chamber.
- Stage 6 Inflation of the radial expansion chamber floating on the endoscope to adjust to the digestive tract.
- Stage 7 Deflation of the radial expansion chamber fixed to the endoscope for its fixing to the digestive tract.
- Stage 8 which is back to stage 3 and successive repetition of the process to move the endoscope forward through the digestive tract.

## Claims

1. Device for automated translational movement of an endoscope through the digestive tract, **characterized in that** it comprises,
(i) translational movement means (1) and
(ii) electronic control means (2), which enable the automated and coordinated method of the translational movement means (1).

2. Device for automated translational movement of an endoscope through the digestive tract according to claim 1, **characterized in that** the translational movement means (1) are pneumatic.

3. Device for automated translational movement of an endoscope through the digestive tract, according to claim 1, **characterized in that** the translational movement means (1) are hydraulically actuated.

4. Device for automated translational movement of an endoscope through the digestive tract, according to claims 1 and 3, **characterized in that** the translational movement means (1) comprise at least,
o a radial expansion chamber immobilized on the endoscope,
o At least one bellows-shaped axial expansion chamber connected via one end to the body of the endoscope
o At least one radial expansion chamber floating on the endoscope and secured to the second end of the bellows-shaped axial expansion chamber.

5. Device for automated translational movement of an endoscope through the digestive tract according to claim 4 **characterized in that** it includes an additional radial expansion chamber at the end of the endoscope.

6. Device for automated translational movement of an endoscope through the digestive tract, according to claims 1 and 3, **characterized in that** the radial expansion chambers (11), (12) and (13), and the bellows-shaped axial expansion chamber (10) have a toroidal shape.

7. Device for automated translational movement of an endoscope through the digestive tract, according to previous claims, **characterized in that** the electronic control means (2) comprise, at least:
• programmable control means (20);
• means of actuation (21) on the translational movement means (1), which enable the activation and deactivation of said translational movement means (1), comprising a plurality of electrovalves;
• pressure collecting means (22);
• control means (23) of the translational movement means (1), so that the outputs of the programmable control means (20) adapt, activating and deactivating the different electrovalves that form the means of actuation (21); and
• man-machine interface means (24) comprising a data display screen (241) and a keyboard (242).

8. Device for automated translational movement of an endoscope through the digestive tract, according to previous claims, **characterized in that** the programmable control means (20) comprise a microcontroller.

9. Device for automated translational movement of an endoscope through the digestive tract, according to previous claims, **characterized in that** the activation and deactivation of the electrovalves of the control means (23) establishes a movement sequence in the translational movement means (1).

10. Method for automated translational movement of the endoscope of the invention with the following stages:
- inserting the endoscope in the digestive tract.
- Inflation of the radial expansion chamber floating on the endoscope to adjust to the digestive tract.
- Inflation of the axial expansion chamber and corresponding displacement of the floating radial expansion chamber together with the digestive tract whereto it was fixed
- Inflation of the radial expansion chamber fixed to the endoscope for its fixing to the digestive tract
- Deflation of the floating radial expansion chamber with the subsequent retraction of the axial expansion chamber and advance of the floating radial expansion chamber.
- Inflation of the radial expansion chamber floating on the endoscope to adjust to the digestive tract.
- Deflation of the radial expansion chamber fixed to the endoscope for its fixing to the digestive tract.
- Back to the previous and successive stages to move the endoscope forward through the digestive tract.

## Amended claims

### Amended claims under Art. 19.1 PCT

1. Device for automated translational movement of an endoscope (4) through the digestive tract, **characterized in that** it comprises,
(i) translational movement means (1), which comprise a radial expansion chamber (11) immobilized on the endoscope (4) and which can be radially expanded and
(ii) electronic control means (2), which enable the automated and coordinated method of the translational movement means (1)
**characterized in that** said translational movement means (1) additionally comprise:
at least one floating radial expansion chamber floating on the endoscope (4) and which can be radially expanded for its fixing to the digestive tract,
at least one bellows-shaped axial expansion chamber (10) connected by a first end to the proximities of the end of advance of the endoscope (4), and which is secured by a second end to the floating radial expansion chamber (12), wherein the electronic control means (2) control the expansion of the axial expansion chamber (10) when the floating radial expansion chamber (12) is fixed to the digestive tract to cause the retraction of the digestive tract and, therefore, the relative advance of the endoscope inside the retracted digestive tract.

2. Device for automated translational movement of an endoscope through the digestive tract according to claim 1, **characterized in that** the translational movement means (1) are pneumatic.

3. Device for automated translational movement of an endoscope through the digestive tract, according to claim 1, **characterized in that** the translational movement means (1) are hydraulically actuated.

4. Device for automated translational movement of an endoscope through the digestive tract according to claim 1, **characterized in that** the end of the endoscope includes an additional radial expansion chamber (13).

5. Device for automated translational movement of an endoscope through the digestive tract, according to claims 1 and 4, **characterized in that** the radial expansion chambers (11, 12, 13), and axial expansion chamber (10) have a toroidal shape.

6. Device for automated translational movement of an endoscope through the digestive tract, according to claim 1, **characterized in that** the electronic control means (2) comprise, at least:
• programmable control means (20);
• means of actuation (21) on the translational movement means (1), which enable the activation and deactivation of said translational movement means (1), comprising a plurality of electrovalves;
• pressure collecting means (22);
• control means (23) of the translational movement means (1), so that the outputs of the programmable control means (20) adapt, activating and deactivating the different electrovalves that form the means of actuation (21) establishing the sequence of movement of the translational movement means (1); and
• man-machine interface means (24) comprising a data display screen (241) and a keyboard (242).

7. Device for automated translational movement of an endoscope through the digestive tract, according to claim 6, **characterized in that** the programmable control means (20) comprise a microcontroller.

8. Method for automated translational movement of an endoscope in the digestive tract, using the automated translational movement device described in any of claims 1 to 7, **characterized in that** it takes place according to the following stages:
- inserting the endoscope in the digestive tract,
- inflation of the floating radial expansion chamber (12) to adjust to the digestive tract,
- inflation of the axial expansion chamber (10) and corresponding displacement of the floating radial expansion chamber (12) together with the digestive tract whereto it was fixed causing the retraction of the digestive tract and, therefore, the relative advance of the endoscope in the retracted digestive tract.
- inflation of the radial expansion chamber (11) fixed to the endoscope for its fixing to the retracted digestive tract, deflation of the floating radial expansion chamber (12) with the subsequent retraction of the axial expansion chamber (10) and displacement of the floating radial expansion chamber (12),
- inflation of the floating radial expansion chamber (12) on the endoscope to adjust to the digestive tract,
- deflation of the radial expansion chamber fixed to the endoscope for its fixing to the digestive tract.
- back to the previous and successive stages to move the endoscope forward through the digestive tract.
